# EUROPÄISCHE PATENTSCHRIFT

(11) **EP 2 568 829 B1**
(45) Veröffentlichungstag und Bekanntmachung des Hinweises auf die Patenterteilung: **19.03.2014**
(21) Anmeldenummer: 11711038.7
(22) Anmeldetag: 23.03.2011
(51) Int. Cl.: A23L 3/00, A23L 3/02, A61L 2/24

(54) **PASTEUR MIT GEREGELTER SPRITZMENGE**
PASTEURIZER WITH CONTROLLED SPRAYER OUTPUT
PASTEURISATION AVEC UNE QUANTITÉ D'INJECTION CONTRÔLÉE

(30) Priorität: 12.05.2010 DE 102010020429
(43) Veröffentlichungstag der Anmeldung: 20.03.2013
(73) Patentinhaber: KHS GmbH, 44143 Dortmund (DE)
(72) Erfinder: RATKE, Andre, 45731 Waltrop (DE); STIENEN, Thomas, 59425 Unna (DE)
(86) Internationale Anmeldenummer: PCT/EP2011/001427
(87) Internationale Veröffentlichungsnummer: WO 2011/141088

(56) Entgegenhaltungen:
- WO-A1-2008/093367
- WO-A2-2008/095576
- DE-A1-102005 028 195
- US-A- 4 441 406
- US-A1- 2002 073 652
- US-A1- 2002 170 440

## Beschreibung

Die Erfindung bezieht sich auf eine Vorrichtung und ein Verfahren zum Behandeln von Gegenständen mit Flüssigkeit, insbesondere auf einen Pasteur und ein Verfahren zum Betrieb eines solchen Pasteurs.

Empfindliche, d.h. zu schnellem Verderb neigende Produkte, beispielsweise auch Flüssigkeiten wie Milch oder Bier, werden durch Pasteurisation, also durch Erwärmung über eine bestimmte Grenztemperatur hinaus haltbar gemacht, wobei bei der Pasteurisation in dem Produkt oder der Flüssigkeit enthaltene, schädliche Keime abgetötet werden.

Um eine ausreichende Pasteurisation des zu pasteurisierenden Füllgutes zu erreichen, muss diesem Füllgut eine bestimmte Anzahl von Pasteurisationseinheiten verabreicht werden. Wird diese Anzahl unterschritten, so ist die Haltbarkeit des Produktes negativ beeinträchtigt, wird diese Anzahl überschritten, so kann es zu geschmacklichen Beeinträchtigungen des Füllgutes kommen, welche ebenfalls überaus unerwünscht sind. Die Anzahl der Pasteurisationseinheiten ist eine Funktion der Ist-Temperatur des Produktes und der Zeitspanne, während der das Produkt diese Ist-Temperatur aufweist.

Pasteure sind allgemein bekannt, die hier relevanten Pasteure dienen der Pasteurisierung einer kontinuierlichen Erzeugniskette. Dazu weist ein solcher Pasteur ein Aufheizgebiet, ein Pasteurisierungsgebiet und ein Kühlgebiet auf, wobei die Produkte auf einem Transportmittel aufstehen, durch welches sie durch die Gebiete in der oben genannten Reihenfolge vom Einlass zum Auslass des Pasteurs transportiert werden, wobei die Produkte in den jeweiligen Gebieten aufgeheizt, pasteurisiert und abgekühlt werden.
Bei dem Transportmittel handelt es sich beispielsweise um einen Gliederkettengurt.

Die einzelnen Behandlungsgebiete sind in Richtung der Fortbewegung der Produkte in Zonen unterteil.

Zum Zwecke der Wärmeübertragung werden die Produkte von oben mit einem temperierten Sprühmedium, beispielsweise Wasser besprüht. Dabei weist das Sprühmedium in den einzelnen Zonen unterschiedliche Temperaturen auf, welche an die jeweils gewünschte Behandlungsart, also Aufwärmen, Pasteurisieren oder Abkühlen der Produkte angepasst ist. Zur Abgabe des Sprühmediums sind oberhalb der Transportebene der Behälter Spritz- oder Sprühdüsen angeordnet, aus denen das Sprühmedium austritt.

Bei Pasteuren nach dem Stand der Technik, wie beispielsweise aus der EP-A-0 437 499 bekannt, erfolgt die Steuerung oder Regelung eines solchen Pasteurs und auch des Pasteurisationsprozesses dadurch, dass die Fördergeschwindigkeit des Transportmittels und/oder die Temperatur des Sprühmediums in mindestens einer Behandlungszone verändert wird.

Ebenfalls bekannt wurde eine Pasteurisationsvorrichtung entsprechend der US 4,441,406. Diese Schrift schlägt einen verbesserten Pasteur vor, welcher sowohl eine Überpasteurisation als auch eine Unterpasteurisation der Produkte sicher vermeiden soll, wobei insbesondere auch ein besonders niedriger Energieverbrauch im Störungsfall realisiert werden soll. Mehr im Detail sieht diese Schrift zum Zwecke der Energieeinsparung die Anordnung eines externen Puffer- oder Speichertanks zur Aufnahme von Sprühflüssigkeit und somit zur Speicherung der, in dieser Sprühflüssigkeit enthaltenen Energie vor.

Ebenfalls bekannt wurde eine Vorrichtung entsprechend der DE 10 2005 028 195 A1. Diese Schrift befasst sich mit einem Pasteur in Tunnelbauweise. Um die Temperatur in den einzelnen Temperaturzonen besser regeln zu können, schlägt diese Schrift vor, die Steuerung und die Verrohrung der Auffangzonen für das Spritzwasser derart zu gestalten, dass zur Temperaturregelung Wasser aus den einzelnen Auffangzonen wahlweise entnehmbar und wahlweise anderen Temperaturzonen zuführbar ist, wobei Wasser in den einzelnen Temperaturzorien getrennt von anderen Temperaturzonen in einem jeweiligen Zonenpuffer gepuffert wird.

Ebenfalls bekannt wurde eine Pasteurisationsvorrichtung entsprechend der WO 2008/095576 A2. Zur Reduzierung des Energiebedarfs von Anlagen zur Pasteurisation von flüssigen Füllgütern in gefüllten und verschlossenen Flaschen oder dergleichen Behälter sieht diese Schrift vor, dass Abwasser, welches am Auslass einer Reinigungs- oder Spülmaschine anfällt, zur Nutzung seiner Inneren Energie oder aber auch zur Nutzung des Abwassers selbst, der Pasteurisationsvorrichtung zugeführt wird.

Allen bekannten Pasteuren ist gemeinsam, dass der, in Kubikmetern pro Stunde und Quadratmeter Behandlungsfläche [m³/(h*m²)] gemessene, Volumenstrom des Sprühmediums zumindest innerhalb jeder einzelnen Behandlungszone entweder Null ist - die Spritzung also abgeschaltet ist -, oder aber konstant bleibt. Dabei ist zu beachten, dass bislang lediglich solche Pasteure bekannt geworden sind, bei denen der Volumenstrom in allen Behandlungszonen eines Pasteurs gleich groß ist.

Durch diese Gegebenheiten haftet dem bekannten Stand der Technik somit der Nachteil an, dass Pasteure bzw. der Pasteurisationsprozess bei Veränderungen des Betriebszustandes, beispielsweise bei störungsbedingten Unterbrechungen, Schwankungen der Anzahl der Produkte je Zeiteinheit usw. lediglich durch die Veränderung der Prozessparameter Transportgeschwindigkeit und Temperatur des Sprühmediums in einzelnen Behandlungszonen verändert oder angepasst werden können, was die Möglichkeiten zur optimalen Einstellung oder Regelung des Pasteurs oder des Pasteurisationsprozesses erheblich einschränkt.

Aufgabe der Erfindung ist es hier Abhilfe und Verbesserung zu schaffen, so dass ein Pasteur, bzw. das Pasteurisationsverfahren in verbesserter Art und Weise an veränderte Betriebszustände angepasst werden kann.

Dazu sieht die vorliegende Erfindung vor, einen Pasteur so auszubilden, dass auch der Volumenstrom des Sprühmediums gesteuert oder geregelt verändert werden kann, wobei der Volumenstrom des Sprühmediums immer größer als Null ist. Weiterhin sieht die vorliegende Erfindung ein entsprechendes Verfahren vor.

Im nachfolgenden wird die Erfindung anhand eines Ausführungsspieles beschreiben.

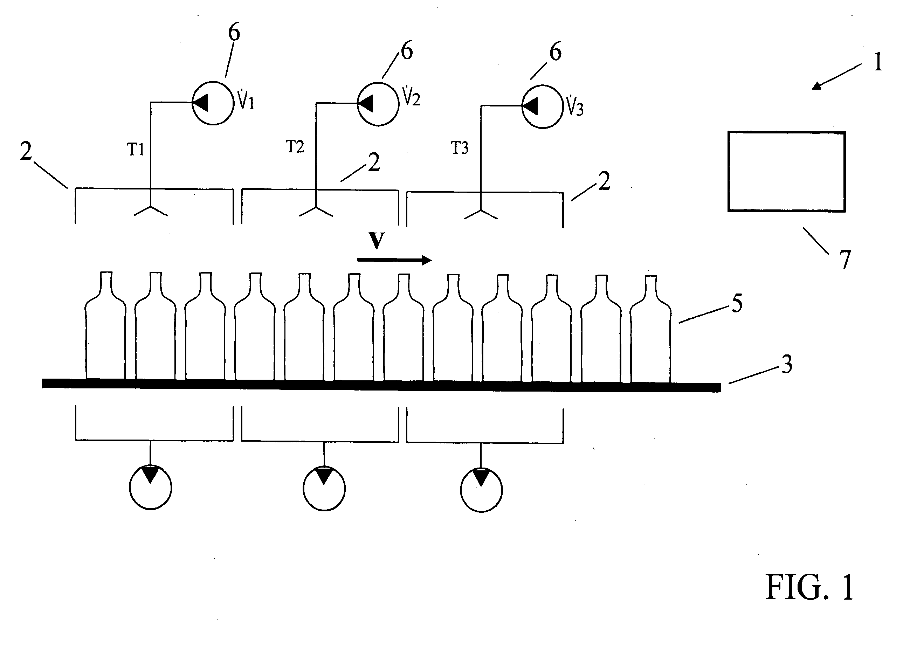

Im Einzelnen zeigen die
- Fig. 1: in einer vereinfachten Darstellung einen erfindungsgemäßen Pasteur, und die
- Fig. 2: eine Veranschaulichung eines besonderen Betriebszustandes eines erfindungsgemäßen Pasteurs.

Bei Versuchen im Hause der Anmelderin konnte festgestellt werden, dass der Wärmeübergang zwischen einem, mit einem Sprühmedium beaufschlagten Behälter 5 und dem Sprühmedium unter anderem auch von dem Volumenstrom des Sprühmediums abhängig ist.
Dabei wurde im Wesentlichen festgestellt, dass mit zunehmendem Volumenstrom des Sprühmediums auch ein zunehmender Wärmeübergang zwischen dem Behälter 5 und dem Sprühmedium auftritt.

Weiterhin wurde dabei allerdings auch festgestellt, dass es eine Untergrenze des Volumenstroms gibt, bei deren Unterschreitung kein, bzw. kein mathematisch abbildbarer insbesondere kein mathematisch vorherbestimmbarer Wärmeaustausch zwischen dem Behälter 5 und dem Sprühmedium mehr stattfindet.

Diese Untergrenze des Volumenstroms ist vereinfacht dargestellt, dann erreicht, wenn der Behälter 5 nicht mehr ständig vollständig von dem Sprühmedium benetzt wird.

Weiterhin wurde festgestellt, dass es eine Obergrenze des Volumenstroms des Sprühmediums gibt, ab der eine weitere Steigerung des Volumenstroms keine weitere Steigerung des Wärmeüberganges zwischen Behälter 5 und Sprühmedium nach sich zieht.

Durch umfangreiche Untersuchungen im Hause der Anmelderin konnten Parameter, Konstanten und Einflussfaktoren bestimmt werden, anhand derer der Wärmeübergang zwischen dem Volumenstrom des Sprühmediums und dem Behälter 5 bei dem oben dargestellten, maximal sinnvollen Volumenstrom mathematisch vorherbestimmt werden kann.

Überraschender Weise konnte durch weitere Untersuchungen im Hause der Anmelderin weiterhin festgestellt werden, dass der Wärmeübergang zwischen dem Sprühmedium und dem Behälter 5 auch für solche Volumenströme mathematisch vorherbestimmbar ist, die zwischen den beiden oben dargestellten Grenzwerten des Volumenstroms des Sprühmediums liegen.

Durch diese Erkenntnis ergibt sich nun erstmals die Möglichkeit, einen Pasteur, bzw. den Pasteurisationsprozess nicht nur durch die Variation der Parameter Transportgeschwindigkeit und Temperatur des Sprühmediums an veränderte Betriebssituationen anzupassen, sondern auch durch die Variation des Volumenstroms des Sprühmediums.

Dabei sei an dieser Stelle ausdrücklich darauf hingewiesen, dass es ebenfalls möglich ist, derartige Anpassungen ausschließlich durch die Variation des Volumenstroms des Sprühmediums vorzunehmen. Ebenfalls ist es möglich, zur Anpassung des Pasteurs 1, bzw. des Pasteurisationsprozesses, die oben genannten und/oder weitere Parameter in beliebigen Kombinationen zu verwenden und zu variieren.

Zur Modellierung des grundlegenden mathematischen Modells des Wärmeüberganges zwischen Sprühmedium und Behälter 5 ist es zwingend erforderlich, für jeden zu pasteurisierenden Behältertypen zu ermitteln, welcher Wärmeübergang sich zwischen diesem Behältertypen und dem Sprühmedium ergibt, bzw. wie sich dieser Wärmeübergang verändert, wenn beispielsweise die Behälterausgangstemperatur T , die Temperatur des Sprühmediums T1, T2, T3 die Transportgeschwindigkeit v und der Volumenstrom des Sprühmediums V°_{1,2,3} unterschiedliche Werte aufweisen.

Es ist also die Änderung des Wärmeüberganges zwischen Sprühmedium und dem speziellen zu pasteurisierenden Behältertypen bei Variation eines, mehrerer oder aller relevanten Parameter zu bestimmen.

Ist die Änderung des Wärmeüberganges bei Variation eines oder mehrerer der oben genannten oder aber auch weiterer Parameter für einen Behältertypen bekannt, so ist es ermöglicht, den Pasteurisationsprozess auch mittels Variation des Volumenstroms V°_{1,2,3} des Sprühmediums anzupassen.

Aufbauend auf den so gewonnen Erkenntnissen wird es ermöglicht, den Pasteurisationsprozess für jeden einzelnen Behälter 5 während seiner Pasteurisation mitzurechnen und somit auch zu überwachen.

Dazu ist es allerdings zwingend erforderlich, dass sich der zu pasteurisierende Behälter 5 zu jedem Zeitpunkt des Pasteurisationsprozesses in einem solchen Zustand befindet, in dem der Wärmeaustausch zwischen Behälter und Umgebung mit hinreichender Genauigkeit berechnet bzw. mitgerechnet werden kann. Befindet sich der Behälter 5 einmal nicht in einem solchen Zustand, kann er also beispielsweise unkontrolliert Wärme mit seiner Umgebung austauschen, ist eine weitere Fortführung der Berechnung des Pasteurisationsprozesses nicht mehr möglich, woraus sich die Gefahr einer unerwünschten Über- oder Unterpasteurisation ergibt. Eine solche Situation kann beispielsweise bei einem Maschinenstopp auftreten, wenn der Behälter 5 bei abgeschaltetem Sprühmedium frei auf dem Transportmittel aufsteht, wobei alle Parameter, welche einen Wärmeaustausch zwischen diesem Behälter und seiner Umgebung bestimmten, beispielsweise Umgebungstemperatur, Luftfeuchtigkeit, Strömungsgeschwindigkeit der Luft usw. nicht bekannt sind.

Folglich muss ein zu pasteurisierende Behälter 5 zu jedem Zeitpunkt des Pasteurisationsprozesses, insbesondere auch im Falle einer Maschinenstörung oder Produktionsunterbrechung usw. definierten Umgebungsbedingungen ausgesetzt sein, damit der Wärmeaustausch zwischen dem Behälter 5 und seiner Umgebung berechenbar bleibt.

Aus dieser Voraussetzung ergibt sich schließlich, dass der Behälter 5 permanent mit einem Sprühmedium einer bekannten Temperatur T_{1,2,3} beaufschlagt werden muss, wobei zum einen auch der Volumenstrom V°_{1,2,3} des Sprühmediums bekannt sein muss und zum anderen der Volumenstrom V°₁,_{2,3} zumindest so groß sein muss, dass der Behälter 5 während des Besprühens permanent vollständig mit Sprühmedium benetzt ist.

Zur Berechnung des Pasteurisationsprozesses wird für jeden Zeitpunkt des Pasteurisationsprozesses der Wärmeübergang zwischen dem Behälter 5 und dem Sprühmedium bestimmt, wobei unter anderem die Ausgangstemperatur des Behälters, die Temperatur des Sprühmediums T_{1,2,3}, der Volumenstrom V°_{1,2,3} des Sprühmediums und beispielsweise auch die Transportgeschwindigkeit v der Behälter berücksichtigt werden.

Unter Berücksichtigung zumindest der Zeitspanne, in der diese Parameter unveränderte Werte aufweisen, werden die Temperaturänderung des Behälters 5, und somit auch seine Temperatur während der Zeitspanne und/oder seine Endtemperatur nach dem Ende der Zeitspanne ermittelt, wodurch auch die somit verabreichten Pasteurisationseinheiten errechenbar sind.

Nimmt zumindest einer der relevanten Parameter einen anderen Wert ein, so wird die Rechnung unter Verwendung der neuen Werte fortgesetzt, wodurch der gesamte Pasteurisationsprozess berechnet wird.

Zur Durchführung des erfindungsgemäßen Verfahrens ist ein Pasteur 1 mit Sensoren ausgestattet, welche den Betriebszustand zumindest des Pasteurs 1 und/oder zumindest eines Teils der gesamten Produktionsanlage überwachen.

Durch die Verwendung geeigneter Sensoren, wie sie dem Fachmann seit langem bekannt sind, werden beispielsweise Informationen über den Belegungs- oder Füllungsgrad des Transportmittels 3 mit Behältern 5, die Temperaturen T_{1,2,3} der Sprühmedien in den einzelnen Behandlungszonen, der Fördergeschwindigkeit v des Transportmittels 3 usw. erfasst.

Diese Informationen werden an eine geeignete Steuervorrichtung 7, beispielsweise eine Vorrichtung zur elektronischen Datenverarbeitung übermittelt.

In dieser Steuervorrichtung 7 sind vorzugsweise auch Soll-Werte und Soll-Pasteurisationsabläufe für alle relevanten zu pasteurisierenden Produkte gespeichert.

Ebenfalls sind vorzugsweise in dieser Steuervorrichtung 7 auch für alle relevanten Behälter- oder Produkttypen alle relevanten Angaben zu den Änderungen des Wärmeüberganges zwischen dem Behälter und dem Sprühmedium in-Abhängigkeit von den relevanten Parametern gespeichert.

Weiterhin ist Steuervorrichtung 7 mit entsprechenden Stellgliedern des Pasteurs 1, wie beispielsweise Pumpen 6, Ventilen, Heizelementen, Antriebsmotoren usw. verbunden, und kann diese in deren Arbeits- oder Wirkungsweise aktiv beeinflussen.

Die Variation bzw. Regelung des Volumenstroms des Sprühmediums erfolgt zumindest bei einer der Behandlungszonen, vorzugsweise jedoch für alle Behandlungszonen, wobei vorzugsweise der Volumenstrom für jede einzelne Behandlungszone separat geregelt oder gesteuert werden kann.

Bevorzugt wird der Volumenstrom des Sprühmediums durch Prozesswasserpumpen, - nachfolgend Hydraulikpumpen genannt - erzeugt, welche z.B. durch frequenzgeregelte Motoren angetrieben werden, da auf diese Weise der Volumenstrom besonders energiesparend erzeugt und auch verändert werden kann.

Ebenfalls kann der Volumenstrom auch durch Stromregelventile und/oder Bypassleitungen und/oder durch in ihrem Abgabevolumenstrom veränderbare Spritzventile beeinflusst werden.

Ebenfalls kann der Volumenstrom auch dadurch verändert werden, dass ein Teil der vorhandenen Spritzventile gesteuert oder geregelt vollständig geöffnet oder geschlossen wird.

Die Steuerung oder Regelung des Pasteurisationsprozesses erfolgt zunächst in der bekannten Art und Weise, wonach in Abhängigkeit des zu pasteurisierenden Produktes das geeignete Pasteurisationsprogramm gewählt wird. Wie aus dem Stand der Technik bekannt, reagiert die Steuerung des Pasteurs eigenständig auf Veränderungen des Betriebszustandes, die beispielsweise von den Sensoren oder aber auch durch aktive manuelle Eingriffe des Bedienungspersonals an die Steuerung gemeldet werden, wobei entsprechend dem Stand der Technik der Pasteurisationsprozess durch Variation der Parameter Transportgeschwindigkeit v und Temperatur T_{1,2,3} der Sprühmedien an den geänderten Betriebszustand angepasst wird.

Als Neuerung gegenüber diesem Stand der Technik kann bei einem erfindungsgemäßen Pasteur 1 auch der Volumenstrom V°_{1,2,3} des Sprühmediums zumindest in einer Behandlungszone 2 variiert werden. Dabei wirkt die Steuerung 7 des Pasteurs 1 in Abhängigkeit des aktuellen Betriebszustandes und der Kenntnis über das Änderungsverhaltens des Wärmeüberganges so auf die beeinflussbaren oder veränderbaren Parameter des Pasteurs 1 ein, dass eine vorteilhafte Anpassung des Pasteurisationsprozesses an den geänderten Betriebszustand erzielt wird.

Dabei können die Veränderungen durch eine Regelung oder aber auch durch eine Steuerung der entsprechenden Parameter, bzw. der sie beeinflussenden Stellglieder, also Pumpen 6 , Ventile, Heizelemente, Antriebsmotoren beeinflusst werden.

Im Nachfolgenden wird die Erfindung an Ausführungsbeispielen näher erläutert.

### Beispiel 1: Produktionsunterbrechung

Kommt es zu einer Produktionsunterbrechung, d.h. zu einem Stillstand des Transportmittels 3 des Pasteurs 1, so ist es, wie oben ausführlich dargestellt, zwingend erforderlich, die Behälter 5 definierten Umgebungsbedingungen auszusetzen. Dieses Erfordernis zieht wiederum nach sich, dass der Volumenstrom des Sprühmediums bei Pasteuren nach dem Stand der Technik unverändert aufrecht erhalten wird, was erhebliche Energiekosten für das permanente Umpumpen und Temperieren eines erheblichen Volumens des Sprühmediums nach sich zieht.

Durch die Anwendung der vorliegenden Erfindung wird es nun erstmals ermöglicht, den Volumenstrom V°_{1,2,3} des Sprühmediums zumindest in einer der Behandlungszonen zu vermindern, wodurch sich die Energiekosten erheblich vermindern lassen.

Wie oben dargestellt, ist es dabei möglich, den Volumenstrom V°_{1,2,3} des Sprühmediums so weit zu vermindern, dass der Behälter gerade eben noch vollständig vom Sprühmedium benetzt wird, ohne dass dabei die mathematische Berechenbarkeit des Pasteurisationsprozesses verloren gehen würde.

### Beispiel 2: Minderleistung

Kommt es zu einer verminderten Zufuhr von Behältern 5, so ist es bei Pasteuren 1 nach dem Stand der Technik üblich, diese entweder unverändert weiter zu betreiben, oder aber vollständig abzuschalten, d.h. so lange keine Behälter 5 in den Pasteur 1 einfahren zu lassen bis wieder eine ausreichende Zahl von Behältern zur Verfügung steht.

Dabei ist es von besonderer Bedeutung, dass der unveränderte Betrieb eines Pasteurs 1 bei verminderter Behälterzufuhr dazu führt dass das Transportmittel 3 nicht vollständig belegt ist, was schließlich zu einem verminderten Wirkungsgrad der Gesamtanlage und zu einem Anstieg der Stückkosten je pasteurisiertem Behälter 5 führt.

Durch die die Anwendung der vorliegenden Erfindung wir es nun ermöglicht, den Pasteur 1 in einer Betriebsart zu betreiben, bei der die Transportgeschwindigkeit und auch der Volumenstrom V°_{1,2,3} des Sprühmedium variiert, beispielsweise gesenkt werden, wodurch in Summe eine vollständige Belegung des Transportmittels 3 und ein, an diese reduzierte Transportgeschwindigkeit v angepasster Pasteurisationsprozess ermöglicht werden.

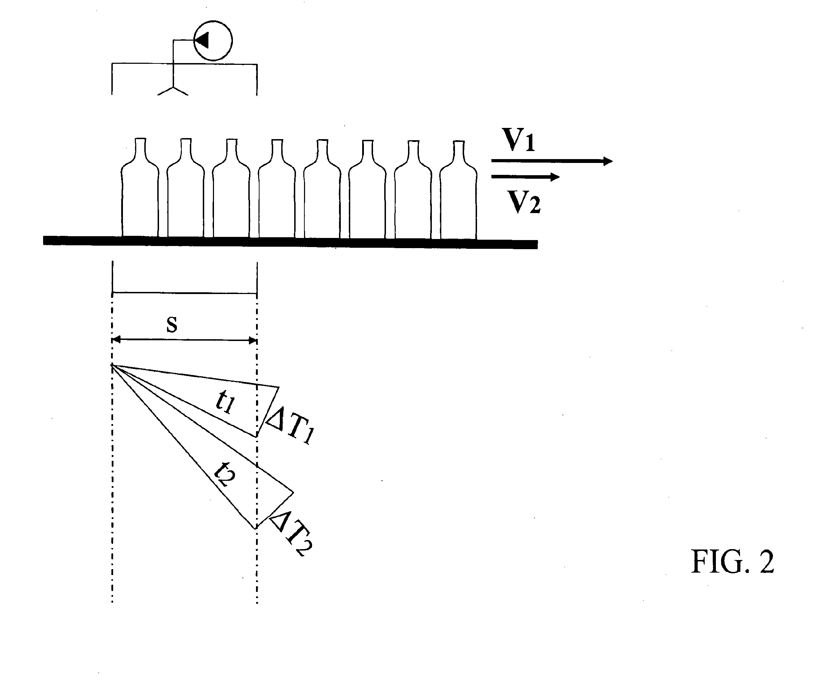

Diese Sachverhalte werden in der Figur 2 dargestellt.

Kommt es zu einer verminderten Bereitstellung von Behältern 5 vor dem Pasteur 1, so wird erfindungsgemäß zunächst die Geschwindigkeit des Transportmittels 3 ausgehend von der Geschwindigkeit v₁ auf die Geschwindigkeit v₂ vermindert, wobei die Geschwindigkeit v₂ so gewählt ist, dass das Transportmittel 3 auch bei der vorliegenden Bereitstellung einer geringeren Anzahl von Behältern 5 vollständig mit Behältern 5 gefüllt ist.

Durch die Reduzierung der Transportgeschwindigkeit v verlängert sich die Zeitspanne, welche für das Durchfahren einer Behandlungszone der Länge s erforderlich ist, von der Zeitspanne t₁ auf die Zeitspanne t₂. Dabei ist es wesentlich, dass die Behälter 5 am Ende der beiden Zeitspannen t₁ und t₂ jeweils die identische Temperaturänderung ΔT aufweisen müssen, da für den Pasteurisationsprozess vorgesehen ist, dass am Ende einer Behandlungszone eine bestimmte Temperatur T erreicht ist. Folglich gilt das Erfordernis ΔT1=ΔT2.

Durch die vorliegende Erfindung kann nun die zusätzlich zur Verfügung stehende Zeit dazu genutzt werden, die Behälter 5 durch eine Reduzierung des Volumenstromes V°_{1,2,3} des Sprühmediums langsamer zu erwärmen, so dass die Soll-Temperaturänderung ΔT2 rechtzeitig aber nicht vorzeitig erreicht wird, wodurch Energieeinsparungen ermöglicht werden.

Entsprechend ist es selbstverständlich auch möglich, den bei der Pasteurisation üblichen Abkühlungsprozess ebenfalls langsamer ablaufen zu lassen.

Analog ist es selbstverständlich auch möglich, den Pasteur 1 bei einer gesteigerten Behälteranlieferung mit erhöhter Geschwindigkeit v zu betreiben, wobei die Behälter 5 durch einen entsprechend gesteigerten Volumenstrom V°_{1,2,3} des Sprühmediums die Soll-Temperaturänderung auch innerhalb der nun verkürzten Zeitspanne erreichen.

Dabei versteht es sich von selbst, dass anstelle einer Soll-Temperaturänderung auch das Erreichen einer Soll-Temperatur vorgesehen werden kann, ohne dass der Schutzbereich der vorliegenden Erfindung verlassen wird.

### Beispiel 3: Unterschiedliche Behältertypen

Ein Pasteur 1 kann prinzipbedingt nur für einen zu pasteurisierenden Behältertypen optimal ausgelegt werden. Ist allerdings vorgesehen, einen solchen Pasteur 1 für die Pasteurisation unterschiedlicher Behältertypen zu verwenden, so muss dieser Pasteur 1 zwingend so ausgelegt werden, dass auch der "Behälter mit dem größten Wärme- und Kühlbedarf und mit dem schlechtesten Wärmeübergang" noch sicher pasteurisiert wird.

Bei der Pasteurisation anderer Behältertypen erfolgt die Anpassung des Pasteurisationsprozesses nach dem Stand der Technik lediglich durch eine Variation der Parameter Transportgeschwindigkeit v und Temperatur T_{1,2,3} der Sprühmedien. Zwar gelingt auch bei einem auf diese Weise angepassten Pasteurisationsprozess in der Regel eine einwandfreie Pasteurisation des Produktes, doch ist dazu häufig ein erhöhter Energieaufwand notwendig, welcher letztlich zu erhöhten Stückkosten führt.

Erfindungsgemäß wird vorgeschlagen, neben oder anstatt der Parameter Transportgeschwindigkeit v und/oder Temperatur T_{1,2,3} des Sprühmediums den Parameter Volumenstrom V°_{1,2,3} des Sprühmediums zu variieren, um einen real ausgeführten Pasteur 1, bzw. einen Pasteurisationsprozess an unterschiedliche Behältertypen anzupassen.

Dabei ist es von besonderem Vorteil, wenn für die Pasteurisation unterschiedlicher Behältertypen für jeden einzelnen dieser unterschiedlichen Behältertypen unterschiedliche Volumenströme V°_{1,2,3} des Sprühmediums vorgesehen sind.

Durch diese Vorgehensweise wird es beispielsweise ermöglicht, einen Behälter 5 mit geringerem Wärme- oder Kühlbedarf und gutem Wärmeübergang mit geringeren Volumenströmen V°_{1,2,3} des Sprühmediums zu behandeln, als einen Behälter 5 mit großem Wärme- oder Kühlbedarf und schlechtem Wärmeübergang.

Dieser Aspekt der Erfindung ist insbesondere dann von Bedeutung, wenn berücksichtigt wird, dass die Größe und die Fördergeschwindigkeit eines Pasteurs im Wesentlichen vom Durchmesser der Behälter und der gewünschten Leistung des Pasteurs [Behälter/Stunde] bestimmt wird, da sich aus den Faktoren Behälterdurchmesser, Anzahl Behälter pro Stunde und Pasteurisationszeit eine Flächengröße oder Flächenbelegung für den Pasteur 1 ergibt.

Durch die, durch die vorliegende Erfindung vorgeschlagene Variation des Volumenstroms V°_{1,2,3} des Sprühmediums ergibt sich eine wesentlich größere Anzahl von Wertepaaren der variierbaren Parameter, bei denen es möglich ist, einen Pasteur 1 so zu betreiben, dass das Transportmittel bei genauer Einhaltung der Pasteurisationszeit auch bei unterschiedlichsten Behälterdurchmessern stets voll belegt ist.

Beispielsweise kann vorgesehen sein, einem kleinen Behälter 5 mit geringem Wärmebedarf und gutem Wärmeübergang bei einer vorgegebenen Leistung des Pasteurs 1 bei niedriger Transportgeschwindigkeit v mit niedrigem Volumenstrom V°_{1,2,3} des Sprühmediums zu beaufschlagen, wohin gegen ein großer Behälter 5 mit großem Wärmebedarf und schlechtem Wärmeübergang bei identischer Leistung bei großer Transportgeschwindigkeit v mit großem Volumenstrom V°_{1,2,3} beaufschlagt wird.

Durch diese Vorgehensweise wird insgesamt eine volle Belegung des Pasteurs 1 auch in unterschiedlichen Behälterdurchmessern erreicht, was letztlich zu niedrigen Pasteurisationskosten je Behälter führt.

### Beispiel 4: Lücke in Produktstrom

Bei bestimmten Betriebssituationen kann es vorkommen, dass die Zufuhr der Behälter 5 zum Pasteur für ein bestimmtes Zeitintervall vollständig unterbrochen ist, so dass das Transportmittel 3 einen Bereich aufweist, welcher keinen Behälter 5 beinhaltet. Bedingt durch die Arbeitsweise eines Pasteur 1 muss eine solche Lücke des Behälterstroms mit derselben Geschwindigkeit v durch den Pasteur 1 gefahren werden, als wenn das Transportmittel 3 vollständig mit Behältern 5 belegt wäre. Bei bekannten Pasteuren 1 wird auf eine solche Lücke ausschließlich dadurch reagiert, dass die Temperatur T_{1,2,3} des Sprühmediums der jeweiligen Behandlungszone 2, in der sich die Lücke jeweils befindet, um eine bestimmte Temperatur abgesenkt wird.

Erfindungsgemäß wird vorgeschlagen, in den Behandlungszonen 2, in denen sich eine solche Lücke befindet, bzw. in solchen Behandlungszonen 2, welche aufgrund einer Lücke im Behälterstrom keine Behälter beinhalten, den Volumenstrom des Sprühmediums zu reduzieren.

Durch diese Reduzierung des Volumenstroms lassen sich die durch Wärmeverluste und das Erzeugen des Volumenstroms verursachten Kosten senken.

Es versteht sich von selbst, dass die in den Ausführungsbeispielen genannten Betriebssituationen und die diesen Betriebssituationen zugeordneten Maßnahmen - auch in der jeweiligen Kombination - lediglich Beispiele darstellen, und den Umfang der vorliegenden Erfindung nicht einschränken. Insbesondere schränken diese Ausführungsbeispiele den Umfang der vorliegenden Erfindung nicht in der Weise ein, dass die genannten Kombinationen von Betriebssituation und Maßnahme zwingend beibehalten werden müssen. Vielmehr erstreckt sich die vorliegende Erfindung auch andere, "1 zu 1"-, "1 zu n"- und "n zu1"-Kombinationen der genannten und weiterer Betriebssituationen und Maßnahmen.

### Bezugszeichen

- 1: Pasteur
- 2: Behandlungszonen
- 3: Transportmittel
- 4: Steuervorrichtung
- 5: Behälter
- 6: Pumpe
- 7: Steuervorrichtung
- T1, T2, T3: Temperatur Sprühmedium
- ΔT1,ΔT2: Temperaturänderungen
- V°_{1,2,3}: Volumenstrom Sprühmedium
- t1, t2: Zeitspannen
- s: Länge Behandlungszone

## Patentansprüche

1. Verfahren für die Pasteurisierung einer kontinuierlichen Erzeugniskette in einer Vorrichtung mit einem Aufheizgebiet, einem Pasteurisierungsgebiet und einem Kühlgebiet, und mit Transportmitteln, welche die Produkte durch die Gebiete in der oben genannten Reihenfolge vom Einlass zum Auslass der Vorrichtung führen, wobei das Aufheizen, Pasteurisieren und Kühlen durch Wärmeübertragung zwischen den Produkten und einem Sprühmedium, vorzugsweise Wasser, mit welchem die Produkte besprüht werden, erreicht werden, wobei die Gebiete in Richtung der Fortbewegung der Produkte in Behandlungszonen unterteilt sind, in denen die Temperatur des Sprühmediums an die gewünschte Folge der Wärmeübertragung in der Zone angepasst ist, **dadurch gekennzeichnet, dass** zur Variation des Pasteurisationsprozesses eine Veränderung des Volumenstromes V°_{1,2,3} des Sprühmediums in mindestens einer Behandlungszone (2) vorgesehen ist, wobei der Volumenstrom V°_{1,2,3} des Sprühmed iums zwischen einem Maximalwert und einem Minimalwert verändert wird.

2. Verfahren nach Anspruch 1, **dadurch gekennzeichnet, dass** zur Variation des Pasteurisationsprozesses zusätzlich die Temperatur des Sprühmediums T_{1,2,3} und/oder die Geschwindigkeit v des Transportmittels (3) veränderbar sind.

3. Verfahren nach Anspruch 1 oder 2, **dadurch gekennzeichnet, dass** für die Pasteurisation unterschiedlicher Behältertypen unterschiedliche Volumenströme V°_{1,2,3} des Sprühmediums vorgesehen sind.

4. Verfahren nach einem der vorhergehenden Ansprüche, **dadurch gekennzeichnet, dass** der Volumenstrom V°_{1,2,3} des Sprühmediums bei bestimmten Betriebsituationen des Pasteurs (1) zumindest in einer Behandlungszone (2) gegenüber dem normalen Volumenstrom vermindert wird.

5. Verfahren nach einem der vorhergehenden Ansprüche 1 bis 3, **dadurch gekennzeichnet, dass** der Volumenstrom V°_{1,2,3} des Sprühmediums bei bestimmten Betriebsituationen des Pasteurs (1) zumindest in einer Behandlungszone (2) gegenüber dem normalen Volumenstrom gesteigert wird,

6. Verfahren nach einem der vorhergehenden Ansprüche, **dadurch gekennzeichnet, dass** eine bestimmte Temperaturänderung eines zu pasteurisierenden Behälters (5) bei einem verminderten Volumenstrom V° des Sprühmediums in einer längeren Zeitspanne erreichbar ist.

7. Verfahren nach einem der vorhergehenden Ansprüche, **dadurch gekennzeichnet, dass** eine bestimmte Temperaturänderung eines zu pasteurisierenden Behälters (5) bei einem gesteigerten Volumenstrom V° des Sprühmediums in einer verkürzten Zeitspanne erreichbar ist.

8. Pasteur (1) für die Pasteurisierung einer kontinuierlichen Erzeugniskette, mit der ein Verfahren nach einem der Ansprüche 1 bis 7 durchgeführt werden kann, wozu die Vorrichtung ein Aufheizgebiet, ein Pasteurisierungsgebiet und ein Kühlgebiet zusammen mit Transportmitteln aufweist, welche die Produkte durch die Gebiete in der oben genannten Reihenfolge vom Einlass zum Auslass der Vorrichtung führen, wobei das Aufheizen, Pasteurisieren und Kühlen durch Wärmeübertragung zwischen den Produkten und einem Sprühmedium, vorzugsweise Wasser, mit welchem die Produkte besprüht werden erreicht werden, wobei die Gebiete in Richtung der Fortbewegung der Produkte in Behandlungszonen (2) unterteilt sind, in denen die Temperatur T1,2,3 des Sprühmediums an die gewünschte Folge der Wärmeübertragung in der Zone angepasst ist, wobei Mittel zur Veränderung des Volumenstroms V°1,2,3 des Sprühmediums in mindestens einer Behandlungszone (2) vorgesehen sind, wobei der Volumenstrom V°1,2,3 der Sprühmediums geregelt oder gesteuert zwischen einem Maximalwert und einem Minimalwert veränderbar ist, **dadurch gekennzeichnet, dass** der Volumenstrom V°1,2,3 des Sprühmediums durch Hydraulikpumpen (6) mit frequenzgeregeltem elektrischen Antriebsmotor oder durch in ihrem Abgabevolumen veränderbare Spritzventile und/oder die Abschaltung eines Teils der Spritzventile veränderbar ist.

## Claims

1. Method for pasteurising a continuous product chain in a device having a heating region, a pasteurisation region and a cooling region, and having transport means which pass the products through the regions in the aforesaid sequence from the inlet to the outlet of the device, wherein the heating, pasteurisation and cooling are achieved by heat transfer between the products and a spray medium, preferably water, with which the products are sprayed, wherein the regions, in the direction of motion of the products, are subdivided into treatment zones in which the temperature of the spray medium is adapted to the desired sequence of the heat transfer in the zone, **characterised in that** for varying the pasteurisation process, a change in the volumetric flow rate V°_{1,2,3} of the spray medium is provided in at least one treatment zone (2), wherein the volumetric flow rate V°_{1,2,3} of the spray medium is changed between a maximum value and a minimum value.

2. Method according to claim 1, **characterised in that**, to vary the pasteurisation process, in addition the temperature of the spray medium T_{1,2,3} and / or the speed v of the transport means (3) can be altered.

3. Method according to claim 1 or 2, **characterised in that**, for the pasteurisation of different container types, different volumetric flow rates V°_{1,2,3} of the spray medium are provided.

4. Method according to any one of the preceding claims, **characterised in that** the volumetric flow rate V°_{1,2,3} of the spray medium in certain operating situations of the pasteuriser (1) is reduced compared to the normal volumetric flow rate at least in one treatment zone (2).

5. Method according to any one of the preceding claims 1 to 3, **characterised in that** the volumetric flow rate V°_{1,2,3} of the spray medium in certain operating situations of the pasteuriser (1) is increased compared to the normal volumetric flow rate at least in one treatment zone (2).

6. Method according to any one of the preceding claims, **characterised in that** a particular temperature change of a container (5) to be pasteurised can be achieved at a reduced volumetric flow rate V° of the spray medium in a longer time span.

7. Method according to any one of the preceding claims, **characterised in that** a particular temperature change of a container (5) to be pasteurised can be achieved at an increased volumetric flow rate V° of the spray medium in a shorter time span.

8. Pasteuriser (1) for pasteurising a continuous product chain, with which a method according to claims 1 to 7 can be carried out, wherein the device has a heating region, a pasteurisation region and a cooling region, together with transport means which pass the products through the regions in the aforesaid sequence from the inlet to the outlet of the device, wherein the heating, pasteurisation and cooling are achieved by heat transfer between the products and a spray medium, preferably water, with which the products are sprayed, wherein the regions, in the direction of motion of the products, are subdivided into treatment zones (2) in which the temperature T1,2,3 of the spray medium is adapted to the desired sequence of the heat transfer in the zone, wherein means to change the volumetric flow rate V°1,2,3 of the spray medium are provided in at least one treatment zone (2), wherein the volumetric flow rate V°1,2,3 of the spray medium can be changed by adjusting or controlling between a maximum value and a minimum value, **characterised in that** the volumetric flow rate V°1,2,3 of the spray medium can be changed by hydraulic pumps (6) with a frequency controlled electric drive motor or by spray valves, with a discharge volume which can be altered, and / or by switching off some of the spray valves.

## Revendications

1. Procédé de pasteurisation d'une chaîne de production continue dans un dispositif doté d'une zone de chauffage, d'une zone de pasteurisation et d'une zone de refroidissement et de moyens de transport qui conduisent les produits en passant par les zones dans l'ordre indiqué ci-dessus de l'entrée à la sortie du dispositif, dans lequel le chauffage, la pasteurisation et le refroidissement sont réalisés par transmission de chaleur entre les produits et un milieu de vaporisation, de préférence, de l'eau avec laquelle les produits sont vaporisés, dans lequel les zones, en direction de la progression des produits, sont subdivisées en zones de traitement dans lesquelles la température du milieu de vaporisation est adaptée à la séquence souhaitée de transmission de chaleur dans la zone, **caractérisé en ce que** pour la variation du processus de pasteurisation, une modification du courant volumique V°_{1, 2, 3} du milieu de vaporisation est prévue dans au moins une zone de traitement (2), le courant volumique V°_{1, 2, 3} du milieu de vaporisation étant modifié entre une valeur maximale et une valeur minimale.

2. Procédé selon la revendication 1, **caractérisé en ce que**, pour la variation du processus de pasteurisation, la température du milieu de vaporisation T _{1, 2, 3} et/ou la vitesse v du moyen de transport (3) sont également modifiables.

3. Procédé selon la revendication 1 ou 2, **caractérisé en ce que**, pour la pasteurisation sont prévus différents types de conteneurs de courants volumiques différents V°_{1, 2, 3} du milieu de vaporisation.

4. Procédé selon l'une des revendications précédentes, **caractérisé en ce que** le courant volumique V°_{1, 2, 3} du milieu de vaporisation est réduit dans certaines situations de fonctionnement du dispositif de pasteurisation (1) au moins dans une zone de traitement (2) par rapport au courant volumique normal.

5. Procédé selon l'une des revendications 1 à 3 précédentes, **caractérisé en ce que** le courant volumique V°_{1,2,3} du milieu de vaporisation est augmenté dans certaines situations de fonctionnement du dispositif de pasteurisation (1) au moins dans une zone de traitement (2) par rapport au courant volumique normal.

6. Procédé selon l'une des revendications précédentes, **caractérisé en ce qu'**une modification déterminée de la température d'un conteneur (5) à pasteuriser avec un courant volumique V° réduit du milieu de vaporisation peut être obtenue en un laps de temps plus long.

7. Procédé selon l'une des revendications précédentes, **caractérisé en ce qu'**une modification déterminée de la température d'un conteneur (5) à pasteuriser avec un courant volumique V° augmenté du milieu de vaporisation peut être obtenue en un laps de temps plus court.

8. Dispositif de pasteurisation (1) pour la pasteurisation d'une chaîne de production continue avec laquelle peut être réalisé un procédé selon l'une des revendications 1 à 7, pour lequel le dispositif présente une zone de chauffage, une zone de pasteurisation et une zone de refroidissement conjointement avec des moyens de transport qui conduisent les produits en passant par les zones dans l'ordre indiqué ci-dessus de l'entrée à la sortie du dispositif, dans lequel le chauffage, la pasteurisation et le refroidissement sont réalisés par transmission de chaleur entre les produits et un milieu de vaporisation, de préférence, de l'eau avec laquelle les produits sont vaporisés, dans lequel les zones, en direction de la progression des produits, sont subdivisées en zones de traitement (2) dans lesquelles la température T1, 2, 3 du milieu de vaporisation est adaptée à la séquence souhaitée de transmission de chaleur dans la zone, dans lequel des moyens de modification du courant volumique V°_{1, 2, 3} du milieu de vaporisation sont prévus dans au moins une zone de traitement (2), dans lequel le courant volumique V°_{1, 2, 3} du milieu de vaporisation est réglé ou commandé de façon modifiable entre une valeur maximale et une valeur minimale, **caractérisé en ce que** le courant volumique V°_{1, 2, 3} du milieu de vaporisation peut être modifié par des pompes hydrauliques (6) avec un moteur d'entraînement électrique à régulation de fréquence ou par des soupapes de pulvérisation modifiables dans leur volume de distribution et/ou la fermeture d'une partie des soupapes de pulvérisation.
